# EUROPÄISCHE PATENTSCHRIFT

(11) **EP 1 824 802 B1**
(45) Veröffentlichungstag und Bekanntmachung des Hinweises auf die Patenterteilung: **17.06.2009**
(21) Anmeldenummer: 05808031.8
(22) Anmeldetag: 20.10.2005
(51) Int. Cl.: C07C 2/40, C07F 15/00

(54) **VERFAHREN ZUR HERSTELLUNG VON UNVERZWEIGTEN ACYCLISCHEN OCTATRIENEN**
METHOD FOR THE PRODUCTION OF UNBRANCHED ACYCLIC OCTACTRIENES
PROCEDE DE PRODUCTION D'OCTATRIENES ACYCLIQUES NON RAMIFIES

(30) Priorität: 16.12.2004 DE 102004060520
(43) Veröffentlichungstag der Anmeldung: 29.08.2007
(73) Patentinhaber: Evonik Oxeno GmbH, 45772 Marl (DE)
(72) Erfinder: JACKSTELL, Ralf, 18106 Rostock (DE); BELLER, Matthias, 18211 Nienhagen (DE); NIERLICH, Franz, 45768 Marl (DE); ORTMANN, Dagmara, 3902 Brig-Glis (CH); SURENDRA, Harkal, 45768 Marl (DE)
(74) Vertreter: Hirsch, Hans-Ludwig
(86) Internationale Anmeldenummer: PCT/EP2005/055419
(87) Internationale Veröffentlichungsnummer: WO 2006/063892

(56) Entgegenhaltungen:
- DD-A1- 107 894
- DE-A1- 10 149 348
- US-A- 3 435 088
- JAFARPOUR L ET AL: "A sterically demanding nucleophilic carbene: 1,3-bis(2,6-diisopropylphenyl)imidazol-2-y lidene). Thermochemistry and catalytic application in olefin metathesis" JOURNAL OF ORGANOMETALLIC CHEMISTRY, ELSEVIER-SEQUOIA S.A. LAUSANNE, CH, Bd. 606, Nr. 1, 14. Juli 2000 (2000-07-14), Seiten 49-54, XP004212112 ISSN: 0022-328X
- VICIU M S ET AL: "N-HETEROCYCLIC CARBENE PALLADIUM COMPLEXES BEARING CARBOXYLATE LIGANDS AND THEIR CATALYTIC ACTIVITY IN THE HYDROARYLATION OF ALKYNES" ORGANOMETALLICS, ACS, WASHINGTON, DC, US, Bd. 23, Nr. 15, 19. Juli 2004 (2004-07-19), Seiten 3752-3755, XP001199986 ISSN: 0276-7333

## Beschreibung

Die Erfindung betrifft die Herstellung von einem oder mehreren unverzweigten acyclischen Octatrien(en) durch Dimerisation von 1,3-Butadien undeinen Katalysator dafür.

Acyclische unverzweigte Octatriene sind Comonomere für die Herstellung von modifiziertem Polyethylen oder Polypropylen. Sie können als Komponente für die Herstellung von Terpolymeren, das sind Elastomere, die durch Polymerisation von Monoolefinen und einem Kohlenwasserstoff mit mindestens zwei Doppelbindungen im molaren Verhältnis 2 zu 1 gewonnen werden, genutzt werden. Darüber hinaus können die unverzweigten acyclischen Octatriene zu den entsprechenden Mono-, Di- oder Triepoxiden umgesetzt werden. Diese Epoxide können beispielsweise Vorstufen für Polyether sein.

Lineare Octatriene können z. B. durch Dimerisierung von 1,3-Butadien hergestellt werden. Bei der Dimerisierung von 1,3-Butadien können höhere Oligomere, cyclische Dimere wie 1,5-Cyclooctadien und Vinylcyclohex-4-en, verzweigte und lineare acyclische Dimere sowie Gemische davon entstehen. Als Katalysator für die Dimerisierung von 1,3-Butadien zu linearen Dimeren werden üblicherweise Komplexe der Metalle der achten Nebengruppe des Periodensystems, beispielsweise die des Eisens, des Kobalts, des Rhodiums, des Nickels oder des Palladiums, eingesetzt.

Nickelkomplexe mit einer trivalenten Phosphorverbindung als Ligand werden z. B. in DD 107 894, DD 102 688 und US 3,435,088 als Katalysatoren eingesetzt. In US 4,593,140 wird ein Nickel-Phosphinit-Komplex als Katalysator eingesetzt, bei dem der OR-Rest eine Aminogruppe enthält.

In US 3,691,249 und DD102 376 wird ein Palladium-Phosphin-Komplex, in US 3,714,284 ein Palladium-Phosphit-Komplex und in DE 16 68 326 Bis(triphenylphosphin)palladiummaleinanhydrid-Komplex als Katalysator verwendet.

Die genannten Verfahren zur Dimerisation von 1,3-Butadien liefern lineare Octatriene zum Teil in sehr hohen Ausbeuten, haben jedoch den Nachteil gemeinsam, dass für ein wirtschaftliches technisches Verfahren die Katalysatorstabilität und/oder -aktivität zu gering und/oder die spezifischen Katalysatorkosten zu hoch sind. Zudem haben die phosphorhaltigen Liganden den Nachteil, dass sie sehr oxidationsempfindlich sind.

Es bestand daher die Aufgabe, ein alternatives Katalysatorsystem bereitzustellen, welches einen oder mehrere der Nachteile der Katalysatorsysteme des Standes der Technik nicht aufweist.

Überraschenderweise wurde nun gefunden, dass 1,3-Butadien in sehr hoher Selektivität mit hoher Raum-Zeit-Ausbeute zu linearen Octatrienen, und zwar fast ausschließlich zum 1,3,7-Octatrien, in Gegenwart eines sekundären Alkohols und einer Base umgesetzt werden kann, wenn als Katalysator ein Komplex eines Metalls der achten Nebengruppe des Periodensystems der Elemente verwendet wird, der als Ligand mindestens ein Carben der Strukturformel L, insbesondere 1,3-Bis-(2,6-diisopropylphenyl)-4,5-dimethyl-2-dehydro-3-hydro-imidazol aufweist. Dieser Befund war nicht zu erwarten, da beispielsweise in DE 101 49 348 für die Telomerisation von Butadien mit einem Alkohol zu 1-Alkoxy-2,7-octadien unter anderen als Katalysator ein Palladium-Carben-Komplex eingesetzt wird, dessen Carbeneinheit ebenfalls ein 1,3,4,5-tetrasubstituiertes 2-Dehydro-3-hydroimidazol ist.

Gegenstand der Erfindung ist demnach ein Verfahren zur Herstellung von linearen Octatrienen, aus 1,3-Butadien oder 1,3-Butadien enthaltenden Kohlenwasserstoffgemischen, welches dadurch gekennzeichnet ist, dass die Dimerisierung des 1,3-Butadien in Gegenwart eines sekundären Alkohols und einer Base erfolgt und dass als Katalysator ein Komplex eines Metalls der achten Nebengruppe des Periodensystems der Elemente verwendet wird, der als Ligand zumindest ein Carben mit der Struktur L 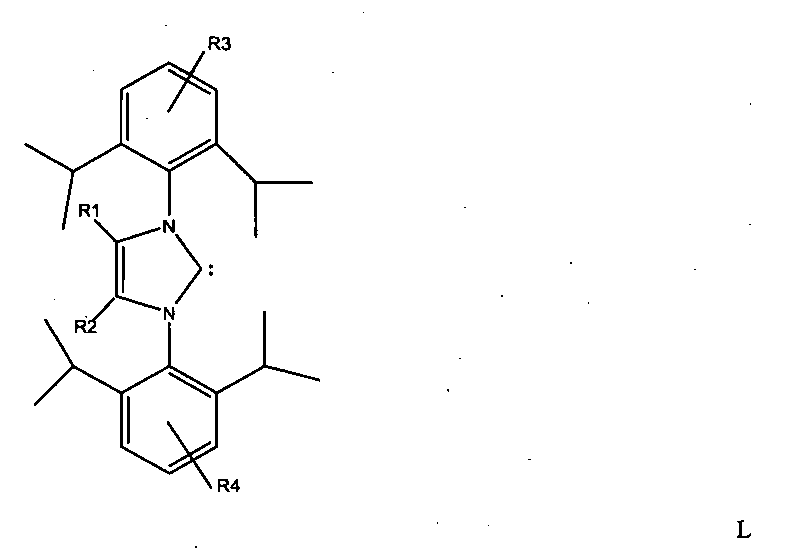 mit R1 und R2 = C₁- bis C₃-Alkylrest und R3 und R4 = H oder ein C₁- bis C₃-Alkylrest, aufweist, wobei die Reste R1 und R2 bzw. R3 und R4 gleich oder unterschiedlich sein können. Die Reste R3 und R4 können in der 3-, 4- oder 5-Stellung mit dem Benzolring verbunden sein. Vorzugsweise sind die Reste R3 und R4 in 4-Stellung an den Benzolring gebunden.

Außerdem ist Gegenstand der vorliegenden Erfindung ein Carbenkomplexkatalysator, welcher sich dadurch auszeichnet, dass der Katalysator ein Ligandenkomplex eines Metalls der achten Nebengruppe des Periodensystems der Elemente ist, der als Ligand zumindest ein Carben mit der Struktur L aufweist, 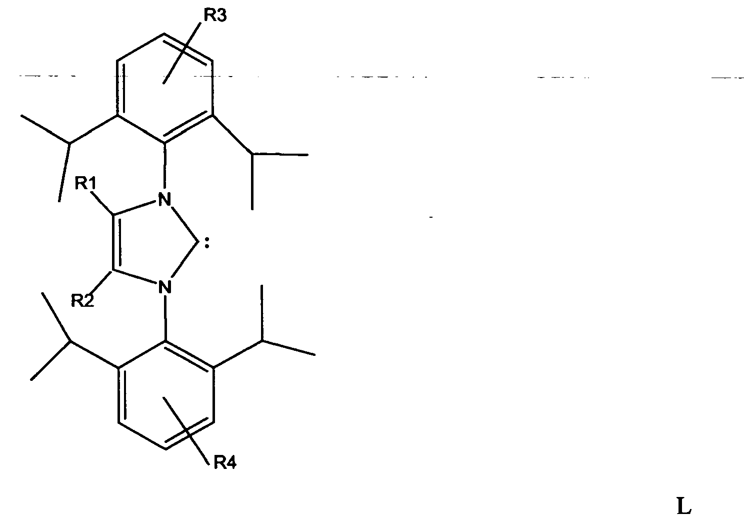 mit R1 und R2 = C₁- bis C₃-Alkylrest und R3 und R4 = H, = C₁- bis C₃-Alkylrest, wobei die Reste R1 und R2 bzw. R3 und R4 gleich oder unterschiedlich sein können.

Die Vorteile des erfindungsgemäßen Verfahrens liegen darin, dass das Reaktionsgemisch wegen der deutlichen Unterschiede in den Siedepunkten leicht in Octatrien, Edukt, sekundäre Alkohole, Nebenprodukte und Katalysator getrennt und der abgetrennte Katalysator zum größten Teil wieder in den Prozess zurückgeführt werden kann. Dadurch ergibt sich ein kostengünstiges Verfahren, weil sowohl der Trennaufwand als auch die Katalysatorkosten gering sind.

Außerdem sind die Vorstufen zu den erfindungsgemäß eingesetzten Liganden problemlos über längere Zeit lagerfähig und die Liganden weniger oxidationsempfindlich. Zudem hat das erfindungsgemäße Verfahren den Vorteil, dass Umsätze an Butadien von über 80 % erhalten werden und auch die Ausbeute an 1,3,7-Octatrien bei über 75 % liegt.

Nachfolgend wird das erfindungsgemäße Verfahren beispielhaft beschrieben, ohne dass die Erfindung, deren Schutzbereich sich aus den Ansprüchen und der Beschreibung ergibt, darauf beschränkt sein soll. Auch die Ansprüche selbst gehören zum Offenbarungsgehalt der vorliegenden Erfindung. Sind im nachfolgenden Text Bereiche bzw. Vorzugsbereiche angegeben, so sollen auch alle in diesen Bereichen liegenden, theoretisch möglichen Teilbereiche und Einzelwerte zum Offenbarungsgehalt der vorliegenden Erfindung gehören, ohne dass diese aus Gründen der besseren Übersichtlichkeit explizit genannt worden sind.

Das erfindungsgemäße Verfahren zur Herstellung von linearen Octatrienen, aus 1,3-Butadien oder 1,3-Butadien enthaltenden Kohlenwasserstoffgemischen, zeichnet sich dadurch aus, dass die Dimerisierung des 1,3-Butadien in Gegenwart eines sekundären Alkohols und einer Base erfolgt und dass als Katalysator ein Komplex eines Metalls der achten Nebengruppe des Periodensystems der Elemente verwendet wird, der als Ligand zumindest ein Carben mit der Struktur L, 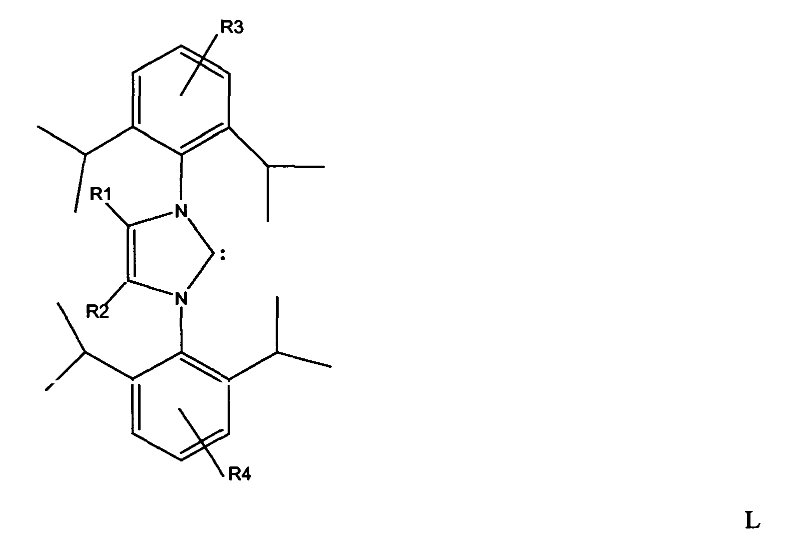 mit R1 und R2 = C₁- bis C₃-Alkylrest und R3 und R4 = H oder ein C₁- bis C₃-Alkylrest aufweist, wobei die Reste R1 und R2 bzw. R3 und R4 gleich oder unterschiedlich sein können. Die Reste R3 und R4 können in der 3, 4 oder 5-Stellung mit dem Benzolring verbunden sein. Vorzugsweise sind die Reste R3 und R4 in 4-Stellung an den Benzolring gebunden. Besonders bevorzugt werden im erfindungsgemäßen Verfahren als Katalysator für die Dimerisierung von 1,3-Butadien zu unverzweigten acyclischen Octatrienen Komplexe der Metalle der achten Nebengruppe des Periodensystems der Elemente verwendet, die mindestens ein 1,3-Bis-(2,6-diisopropylphenyl)-4,5-dimethyl-2-dehydro-3-hydro-imidazol (Struktur L1) als Liganden aufweisen.

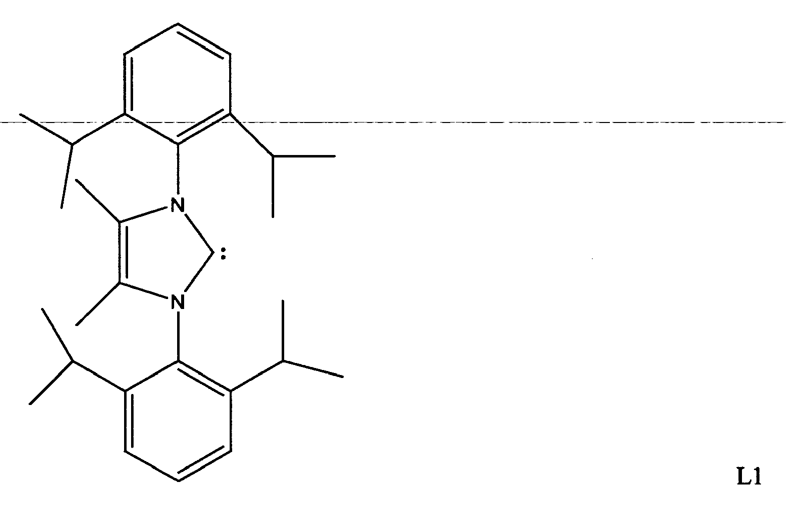

Die im erfindungsgemäßen Verfahren eingesetzten (Komplex-)Katalysatoren können als Katalysatormetall eines oder mehrere der Metalle der VIII Nebengruppe des Periodensystems der Elemente aufweisen. Vorzugsweise weisen die erfindungsgemäß eingesetzten Katalysatoren als Metalle Nickel oder Palladium, besonders bevorzugt Palladium auf.

Es kann vorteilhaft sein, wenn die Dimerisierung des 1,3-Butadiens zu linearen Octatrienen in Gegenwart von zumindest einem weiteren Liganden durchgeführt wird. Dabei ist es zweckmäßig solche Liganden zusätzlich einzusetzen, die die Reaktionsgeschwindigkeit erhöhen, die Selektivität der Bildung von linearen Octatrienen verbessern, die Katalysatorstandzeit verlängern oder andere Vorteile bewirken. Besonders bevorzugt kann das erfindungsgemäße Verfahren zur Dimerisierung von 1,3-Butadien durchgeführt werden, wenn neben dem Carben-Liganden L zumindest 1,1,3,3-Tetramethyl-1,3-Divinyldisiloxan (DVDS) als weiterer Ligand vorhanden ist. Das Verhältnis der optional vorliegenden weiteren Liganden, insbesondere des DVDS, zu dem Carbenliganden L beträgt vorzugsweise von 0,1 zu 1 bis 10 zu 1, bevorzugt von 0,5 zu 1 bis 1,5 zu 1, besonders bevorzugt von 0,9 zu 1 bis 1,1 zu 1 und ganz besonders bevorzugt 1 zu 1.

In den Carbenkomplexen gemäß der Erfindung kann das Metall, insbesondere das Palladium bevorzugt in den Oxidationsstufen 0 und 2 vorliegen. Beispiele für solche Carbenkomplexe sind unter anderem Palladium(0)carben-olefin-Komplexe, Palladiumcarbenphosphinkomplexe, Palladium(0)dicarbenkomplexe, Palladium(2)dicarbenkomplexe, Palladium(0)-carbendienkomplexe, Palladium(2)carbendienkomplexe und Palladium(0)Carben L-DVDS-Komplexe.

Die im erfindungsgemäßen Verfahren eingesetzten Carbenkomplexe können auf verschiedene Weise hergestellt werden. Ein einfacher Weg ist beispielsweise die Anlagerung des Carbens L an eine Metallverbindung, insbesondere eine Palladiumverbindung oder der Austausch eines Liganden von einem Metallkomplex gegen das Carben mit der Struktur L.

Als Vorstufen für die Katalysatoren können insbesondere Metallsalze, vorzugsweise Salze von organischen Säuren oder Halogenwasserstoffsäuren eingesetzt werden. Als Vorstufen für die Palladium aufweisenden Katalysatoren können Palladiumsalze, wie beispielsweise Palladium(II)acetat, Palladium(II)chlorid, Palladium(II)bromid, Lithiumtetrachloropalladat, Palladium(II)acetylacetonat, Palladium(0)-dibenzylidenaceton-Komplexe, Palladium(II)propionat, Palladium(II)chloridbisacetonitril, Palladium(II)-bistriphenylphosphandichlorid, Palladium(II)chloridbisbenzonitril, Bis(tri-o-tolylphosphin)palladium(0) und weitere Palladium(0)- und Palladium(II)-Komplexe eingesetzt werden.

Als Vorstufen für die Nickel aufweisenden Katalysatoren können Nickelverbindungen, wie beispielsweise [Ni(1,5-C₈H₁₂)₂], (c-C₅H₅)₂Ni, (Ph₂P(CH₂)₃PPh₂)₂NiCl₂, (PPh₃)₂NiBr, PPh₃Ni(CO)₂, Nickel(II)acetylacetonat, Nickel(II)Chlorid oder ähnliche, in Katalogen von Chemiehändlern aufgeführte Nickelverbindungen eingesetzt werden

Als Vorstufen für die Palladium aufweisenden Katalysatoren können Palladiumverbindungen, wie beispielsweise Palladium(II)-bistriphenylphosphandichlorid, Palladium(II)chloridbisbenzonitril, Bis(tri-o-tolylphosphin)palladium(0) und weitere Palladium(0)- und Palladium(II)-Komplexe eingesetzt werden.

Das Carben L kann als solches oder als Metallkomplex eingesetzt werden oder aber in situ aus einer Vorstufe erzeugt werden. Das Carben mit der Struktur L und der daraus abgeleitete Metallkomplex kann in situ aus einem Imidazoliumsalz der allgemeinen Struktur S 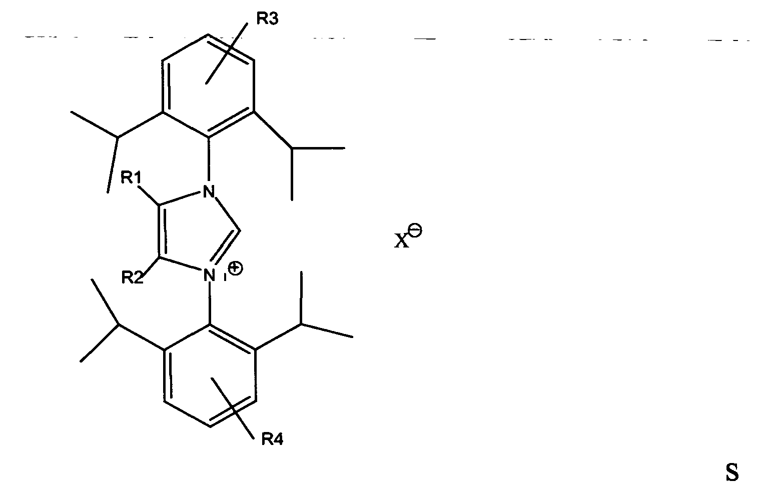 mit R1 und R2 = C₁- bis C₃-Alkylrest und R3 und R4 = H oder ein C₁- bis C₃-Alkylrest, wobei die Reste R1 und R2 bzw. R3 und R4 gleich oder unterschiedlich sein können, und einer Base, insbesondere einer Metallverbindung erzeugt werden. Das besonders bevorzugt eingesetzte Carben 1,3-Bis-(2,6-diisopropylphenyl)-4,5-dimethyl-2-dehydro-3-hydro-imidazol und der daraus abgeleitete Metallkomplex kann beispielsweise in situ aus einem 1,3-Bis-(2,6-diisopropylphenyl)-4,5-dimethyl-3-hydro-imidazoliumsalz mit der allgemeinen Struktur S1 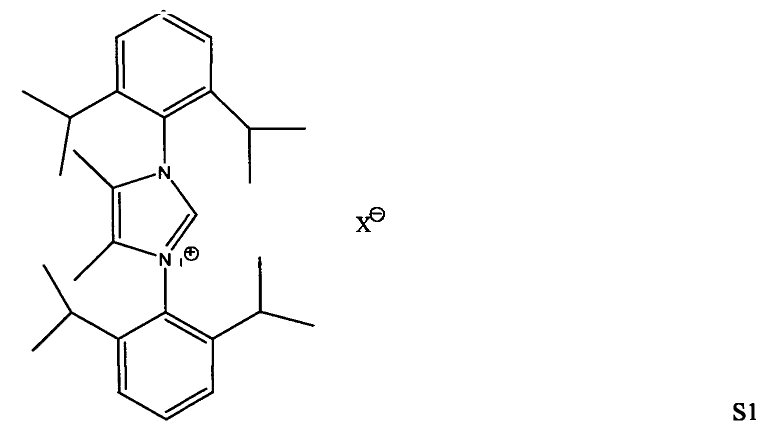 und einer entsprechenden Base, insbesondere einer entsprechenden Metallverbindung erzeugt werden.

Beispiele für X⁻ sind Halogenide, Hydrogensulfat, Sulfat, Sulfonate, Alkylsulfate, Arylsulfate, Borate, Hydrogencarbonat, Carbonat, Alkylcarboxylate, Phosphate, Phosphonate oder Arylcarboxylate. Aus den Salzen mit der Struktur S bzw. S1 kann das Carben L bzw. L1 vorzugsweise durch Umsetzung mit einer Base freigesetzt werden. Die Vorstufen können auf bekannt Weise durch Umsetzung von entsprechend substituierten Anilinen mit entsprechend substituierten 2,3-Butandion und Formaldehyd erhalten werden. Die Herstellung solcher Vorstufen wird beispielsweise in "Nucleophilic Carbenes and their Applications in modern Complex Catalysis, Anthony J. Arduengo und Thomas Bannenberg, The Strem Chemiker, June 2002, Vol. XVIV No. 1" beschrieben.

Wird der Katalysator in situ aus einer Palladiumverbindung und einer Carbenvorstufe der Struktur S hergestellt, wird als Base vorteilhaft ein Alkoholat des bei der Dimerisierung des 1,3-Butadiens verwendeten sekundären Alkohols eingesetzt. Wird beispielsweise Isopropanol als Lösungsmittel verwendet, ist es zweckmäßig Isopropylate als Base einzusetzen. Vorzugsweise werden Alkalialkoholate, insbesondere Natriumalkoholate, verwendet. Optional können anstelle der Alkoholatlösungen auch Lösungen aus Alkalimetallhydroxid und Alkohol verwendet werden.

Im erfindungsgemäßen Verfahren beträgt die Konzentration des Katalysators, formal angegeben in ppm (Masse) an Metall bezogen auf die Gesamtmasse, vorzugsweise von 0,01 ppm bis 1000 ppm, bevorzugt von 0,5 bis 100 ppm und besonders bevorzugt von 1 bis 50 ppm. Das Verhältnis (Mol/Mol) von Carben L zu Metall kann von 0,01/1 bis 250/1, bevorzugt von 1/1 bis 100/1 und besonders bevorzugt von 1/1 bis 50/1 betragen.

Im erfindungsgemäßen Verfahren wird die 1,3-Butadiendimerisierung vorzugsweise in Gegenwart eines sekundären Alkohols mit 3 bis 20 C-Atomen durchgeführt. Der eingesetzte Alkohol kann alicyclisch oder aliphatisch sein. Bevorzugt werden sekundäre aliphatische Alkohole, insbesondere lineare Alkohole verwendet. Es können auch Gemische aus zwei oder mehreren Alkoholen eingesetzt werden. Weiterhin können auch mehrwertige, sekundäre Alkohole, wie z. B. Diole, wie beispielsweise 2,4-Dihydroxypentan, Triole, Tetraole etc., eingesetzt werden. Bevorzugt eingesetzt Alkohole sind Isopropanol und Cyclohexanol.

Besonders bevorzugt wird im erfindungsgemäßen Verfahren Isopropanol eingesetzt.

Das Massenverhältnis von Alkohol zu 1,3-Butadien kann im Bereich von 1/20 bis 20/1, vorzugsweise im Bereich von 4/1 bis 1/2 liegen. Da die Umsetzung in homogener flüssiger Phase erfolgen sollte, bestehen in diesen Bereichen nur dann Einschränkungen, wenn 1,3-Butadien bzw. das eingesetzte 1,3-Butadien-haltige Kohlenwasserstoffgemisch eine Mischungslücke mit dem Alkohol aufweist.

Optional kann das Reaktionsgemisch weitere Lösungsmittel enthalten, wie beispielsweise einen Hochsieder, in dem sich der Katalysator und gegebenenfalls die eingesetzte Base löst.

Erfindungsgemäß erfolgt die Dimerisierung des 1,3-Butadien zu linearen Octatrienen in Gegenwart von freier Base (Base, die nicht für die Erzeugung des Carbens L gebraucht wird). Als Base werden vorzugsweise Alkoholate, besonders bevorzugt Alkoholate des eingesetzten Alkohols verwendet. Besonders bevorzugt werden Alkalimetallalkoholate, insbesondere Natriumalkoholat als Base eingesetzt. Es ist aber auch möglich Alkalimetallhydroxide, wie z. B. NaOH oder KOH, als Basen im erfindungsgemäßen Verfahren einzusetzen.

Das Verhältnis von Base zu 1,3-Butadien beträgt vorzugsweise von 0,01 Mol bis 10 Mol pro 100 Mol 1,3-Butadien, insbesondere von 0,1 Mol bis 5 Mol und ganz besonders bevorzugt von 0,2 Mol bis 1 Mol pro 100 Mol 1,3-Butadien.

Die Temperatur, bei der die Dimerisierung des 1,3-Butadiens zu linearen Octatrienen durchgeführt werden kann, beträgt vorzugsweise von 10 bis 180 °C, bevorzugt von 40 bis 100 °C und besonders bevorzugt von 40 bis 80 °C. Der Reaktionsdruck beträgt vorzugsweise von 0,1 bis 30 MPa, bevorzugt von 0,1 bis 12 MPa, besonders bevorzugt von 0,1 bis 6,4 MPa und ganz besonders bevorzugt von 0,1 bis 2 MPa.

Die Dimerisierung des 1,3-Butadiens kann kontinuierlich oder diskontinuierlich betrieben werden und ist nicht auf den Einsatz bestimmter Reaktortypen begrenzt. Beispiele für Reaktoren, in denen die Dimerisierung durchgeführt werden kann, sind Rührkessel, Rührkesselkaskade, Strömungsrohr und Schlaufenreaktor. Auch Kombinationen verschiedener Reaktoren sind möglich, beispielsweise ein Rührkessel mit nachgeschaltetem Strömungsrohr.

Die Dimerisierung kann, um eine hohe Raum-Zeit-Ausbeute zu erhalten, nicht bis zum vollständigem Umsatz des 1,3-Butadiens durchgeführt werden. Es ist zweckmäßig, den Umsatz auf maximal 95 %, bevorzugt auf maximal 90 % zu begrenzen.

Ausgangsstoff für das erfindungsgemäße Verfahren können reines 1,3-Butadien oder 1,3-Butadien aufweisende Kohlenwasserstoffströme, bevorzugt 1,3-Butadien-reiche Kohlenwasserstoffströme sein. Als Edukt kann insbesondere ein Butadien-haltiger C₄-Schnitt eingesetzt werden. Neben dem 1,3 Butadien können die eingesetzten Kohlenwasserstoffströme unter anderem allenisch ungesättigte Verbindungen enthalten. Als Kohlenwasserstoffstrom kann insbesondere ein C₄-Kohlenwasserstoffschnitt eingesetzt werden. Die Kohlenwasserstoffströme können vorzugsweise z. B. Mischungen von 1,3-Butadien mit anderen C₄- und C₃- oder C₅-Kohlenwasserstoffen sein. Solche Mischungen fallen beispielsweise bei Spalt(Crack)-Prozessen zur Produktion von Ethylen und Propylen an, in denen Raffineriegase, Naphtha, Gasöl, LPG (liquified petroleum gas), NGL (natural gas liquid) usw. umgesetzt werden. Die bei den Prozessen als Nebenprodukt anfallenden C₄-Schnitte können neben 1,3-Butadien, Monoolefine (1-Buten, cis-But-2-en, trans-But-2-en, Isobuten), gesättigte Kohlenwasserstoffe (n-Butan, Isobutan), acetylenisch ungesättigte Verbindungen (Ethylacetylen, Vinylacetylen, Methylacetylen (Propin)) sowie allenisch ungesättigte Verbindungen (hauptsächlich 1,2-Butadien) enthalten. Weiterhin können diese Schnitte geringe Mengen an C₃- und C₅-Kohlenwasserstoffen enthalten. Die Zusammensetzung der C₄-Schnitte ist abhängig vom jeweiligen Spaltverfahren, den Betriebsparametern und dem Einsatzstoff. Die Konzentrationen der einzelnen Komponenten liegen typischerweise in folgenden Bereichen:

| Komponente | Massen-% |
|---|---|
| 1,3- Butadien | 25 - 70 |
| 1-Buten | 9 - 25 |
| 2-Butene | 4 - 20 |
| Isobuten | 10-35 |
| n-Butan | 0,5 - 8 |
| Isobutan | 0,5 - 6 |
| Σ acetylenischer Verbindungen | 0,05 - 4 |
| 1,2-Butadien | 0,05-2 |

Im erfindungsgemäßen Verfahren werden vorzugsweise Kohlenwasserstoffgemische mit einem 1,3-Butadiengehalt von größer 35 Massen-% eingesetzt.

Die Einsatzkohlenwasserstoffe können häufig Spuren von Sauerstoff-, Stickstoff-, Schwefel-, Halogen-, insbesondere Chlor- und Schwermetallverbindungen aufweisen, die im erfindungsgemäßen Verfahren störend sein könnten. Es ist daher vorteilhaft, diese Stoffe zunächst abzutrennen. Störende Verbindungen können z. B. Stabilisatoren, wie z. B. tert.-Butylbrenzcatechin (TBC), oder Kohlendioxid oder Carbonylverbindungen, wie z. B. Aceton oder Acetaldehyd sein.

Die Abtrennung dieser Verunreinigungen kann z. B. durch Wäschen, insbesondere mit Wasser oder wässrigen Lösungen, oder über Adsorber erfolgen.

Durch eine Wasserwäsche können hydrophile Komponenten aus dem Kohlenwasserstoffgemisch ganz oder teilweise entfernt werden, beispielsweise Stickstoffkomponenten. Beispiele für Stickstoffkomponenten sind Acetonitril oder N-Methylpyrrolidon (NMP). Auch Sauerstoffverbindungen können zum Teil über eine Wasserwäsche entfernt werden. Die Wasserwäsche kann direkt mit Wasser durchgeführt werden oder aber mit wässrigen Lösungen, die z. B. Salze wie z. B. NaHSO₃ aufweisen können (US 3,682,779, US 3,308,201, US 4,125,568, US 3,336,414 oder US 5,122,236).

Es kann vorteilhaft sein, wenn das Kohlenwasserstoffgemisch nach der Wasserwäsche einen Trocknungsschritt durchläuft. Die Trocknung kann nach den im Stand der Technik bekannten Verfahren durchgeführt werden. Bei Vorliegen von gelöstem Wasser kann die Trocknung z. B. unter Verwendung von Molekularsieb als Trocknungsmittel oder durch azeotrope Destillation durchgeführt werden. Freies Wasser kann z. B. durch Phasentrennung, z. B. mit einem Koaleszer abgetrennt werden.

Adsorber können eingesetzt werden, um Verunreinigungen im Spurenbereich zu entfernen. Dies kann beispielsweise deshalb vorteilhaft sein, weil im zweiten Prozessschritt Edelmetallkatalysatoren zum Einsatz kommen, die bereits auf Spuren von Verunreinigungen mit deutlicher Aktivitätsabnahme reagieren. Oftmals werden Stickstoff- oder Schwefelverbindungen aber auch TBC über vorgeschaltete Adsorber entfernt. Beispiele für Adsorber sind Aluminiumoxide, Molekularsiebe, Zeolithe, Aktivkohle oder mit Metallen imprägnierte Tonerden (z. B. US 4,571,445 oder WO 02/53685). Adsorber werden von diversen Firmen vertrieben, beispielsweise von der Firma Alcoa unter dem Namen Selexsorb^{®}, von UOP oder von Axens, z. B. mit den Produktserien SAS, MS, AA, TG, TGS oder CMG.

Enthält der eingesetzte Kohlenwasserstoffstrom einen Anteil von größer 100 Massen-ppm an acetylenisch ungesättigten so kann es vorteilhaft sein, wenn aus dem Kohlenwasserstoffstrom, der zunächst gereinigt worden sein kann, im erfindungsgemäßen Verfahren in einem vorgeschalteten Schritt zunächst die acetylenisch ungesättigten Verbindungen bis auf einen Gehalt von kleiner-gleich 100 Massen-ppm, bevorzugt kleiner-gleich 50 Massen-ppm und besonders bevorzugt kleiner-gleich 20 Massen-ppm abgetrennt bzw. entfernt werden, bevor der Kohlenwasserstoffstrom in der Dimerisierungsstufe eingesetzt wird. Das Abtrennen/Entfernen kann z. B. durch Extraktion oder Hydrierung der acetylenisch ungesättigten Verbindungen erfolgen. Gegebenenfalls vorhandenes Methylacetylen kann auch destillativ entfernt werden.

Die Abtrennung der acetylenischen Verbindungen durch Extraktion ist lange bekannt und ist als Aufarbeitungsschritt ein integraler Bestandteil der meisten Anlagen, die 1,3-Butadien aus Crack-C₄ gewinnen. Ein Verfahren zur extraktiven Abtrennung von acetylenisch ungesättigten Verbindungen aus Crack-C₄, wird beispielsweise in Erdöl und Kohle-Erdgas-Petrochemie vereinigt mit Brennstoffchemie Bd. 34, Heft 8, August 1981, Seite 343 - 346 beschrieben. Bei diesem Verfahren werden in einer ersten Stufe durch Extraktivdestillation mit wasserhaltigem N-Methylpyrrolidon (NMP) die mehrfach ungesättigten Kohlenwasserstoffe sowie die acetylenisch ungesättigten Verbindungen von den Monoolefinen und gesättigten Kohlenwasserstoffen abgetrennt. Aus dem NMP Extrakt werden die ungesättigten Kohlenwasserstoffe destillativ abgetrennt und aus dem Kohlenwasserstoffdestillat durch eine zweite Extraktivdestillation mit wasserhaltigem NMP die acetylenisch ungesättigten Verbindungen mit 4-C-Atomen abgetrennt. Bei der Aufarbeitung von Crack-C₄ wird durch zwei weitere Destillationen reines 1,3-Butadien abgetrennt, wobei als Nebenprodukte Methylacetylen und 1,2-Butadien anfallen. Im Rahmen des erfindungsgemäßen Verfahrens kann das auf diese Weise gewonnene 1,3-Butadien Einsatzstoff für die Dimerisation sein.

Die durch Extraktion erhaltenen, 1,3-Butadien aufweisenden Kohlenwasserstoffströme, die gegebenenfalls 1,2-Butadien und/oder weniger als 100 Massen-ppm an acetylenischen Verbindungen enthalten, können direkt oder nach einer Aufarbeitung, vorzugsweise direkt als Edukt in der Dimerisierung verwendet werden.

Vorzugsweise erfolgt die Entfernung der acetylenisch ungesättigten Verbindungen aus dem eingesetzten Kohlenwasserstoffstrom durch Hydrierung der acetylenisch ungesättigten Verbindungen. Um die Ausbeuteverluste, vor allem die an 1,3-Butadien zu vermeiden, muss das Hydrierverfahren sehr selektiv sein, d. h., die Hydrierung von 1,3-Butadien zu linearen Butenen und die Hydrierung von Butenen zu Butanen muss möglichst weitgehend vermieden werden. Für die selektive Hydrierung von acetylenischen Verbindungen in Gegenwart von Dienen und Monoolefine können z. B. kupferhaltige Katalysatoren verwendet werden. Ebenfalls können Katalysatoren, die ein Edelmetall der VIII Gruppe des Periodensystems der Elemente, insbesondere Palladium aufweisen oder Mischkatalysatoren eingesetzt werden. Besonders bevorzugt werden kupferhaltige Katalysatoren oder Katalysatoren, die sowohl Palladium als auch Kupfer aufweisen, verwendet.

Aus den Butadien-haltigen Einsatzstoffen können acetylenische Verbindungen statt durch Hydrierung auch durch Umsetzung mit einem Alkohol entfernt werden. Entsprechende Verfahren sind beispielsweise in US 4 393 249 und DD 127 082 beschrieben. Dabei kann es vorteilhaft sein, bei der Abtrennung der acetylenischen Verbindungen den gleichen Alkohol wie bei der Dimerisierung einzusetzen.

Der Reaktionsaustrag aus der erfindungsgemäßen Dimerisierung kann z. B. lineare Octatriene als Hauptbestandteile sowie Nebenprodukte, "inerte C₄-Kohlenwasserstoffe", Restmengen an 1,3-Butadien, sekundären Alkohol und Katalysatorsystem (Metallkomplex, Liganden, Basen etc.) bzw. dessen Folgeprodukten und gegebenenfalls zugesetztes Lösemittel aufweisen. Je nach Ausgangsstoffgemisch kann im Reaktionsaustrag auch 1,2-Butadien enthalten sein. Die im Austrag der Dimerisierung gegebenenfalls vorhandenen Allene, insbesondere das 1,2-Butadien, können durch Destillation abgetrennt werden.

Die Auftrennung des Austrags der erfindungsgemäßen Dimerisierung kann ganz allgemein nach bekannten technischen Verfahren wie beispielsweise Destillation oder Extraktion erfolgen. Es kann beispielsweise eine destillative Trennung in folgende Fraktionen erfolgen:
eine C₄-Fraktion, die n-Butan, Isobutan, 1-Buten, 2-Butene, Isobuten, 1,3-Butadien, 1,2-Butadien, und gegebenenfalls einen Teil des Alkohols enthält,
eine Fraktion mit den linearen Octatrienen
eine Fraktion mit dem Alkohol
eine Fraktion mit Nebenprodukte
eine Fraktion mit dem Katalysator
gegebenenfalls eine Lösemittelfraktion

Die Fraktion mit dem Alkohol, die Fraktion mit dem Lösemittel sowie die Fraktion mit dem Katalysator bzw. Katalysatorsystem kann jeweils ganz oder teilweise in die Dimerisierung zurückgeführt oder einer Aufarbeitung zugeführt werden.

Das Zielprodukt, also das gemäß dem erfindungsgemäßen Verfahren hergestellte Gemisch von (linearen) Octatrienen, besteht vorzugsweise hauptsächlich, also zu zumindest 90 Massen-%, aus 1,3,7-Octatrien. Die beiden Isomere (cis und trans) des 1,3,7-Octatrien können z. B. im Verhältnis von ca. 1/1,7 vorliegen. Das gewonnene Octatrien kann für die Herstellung der in der Einleitung genannten Produkte, insbesondere zur Herstellung von linearen Octenen verwendet werden. Darüber hinaus kann das Octatrien mit Dienophilen zu den entsprechenden Diels-Alder-Produkten umgesetzt werden, die zur Modifizierung von Polyolefinen oder Polyester genutzt werden können. Weiterhin kann 1,3,7-Octatrien als eine Zwischenstufe für die Herstellung von 1-Octen eingesetzt werden, die analog der Herstellung von 1-Octen aus 1,3,6-Octrätrien erfolgen kann. Dabei wird ausgehend von 1,3,6-Octatrien 1-Octen über folgenden Syntheseweg hergestellt:
- Bildung eines Diels-Alder-Addukts aus Anthracen und 1,3,6-Octatrien, wobei die endständige Doppelbindung des Octatriens reagiert
- Hydrierung der beiden Doppelbindungen in der Seitenkette des Addukts
- Spaltung des hydrierten Addukts (Retro-Diels-Alder-Reaktion) zu Anthracen und 1-Octen

Über diesen Syntheseweg, der in Research Disclosures: "Process for preparing 1-Octene from Butadiene", Heft 476, Dezember 2003, Seite 1281 beschrieben wird, kann auf einfache Weise aus Butadien über die Octatriene 1-Octen hergestellt werden.

Weiterhin kann das erfindungsgemäß hergestellte lineare Octatrien als Vorstufe für die Herstellung linearer Octene sein, die durch Selektivhydrierung hergestellt werden können. Diese sind ihrerseits begehrte Zwischenstufen für die Herstellung von wenig verzweigten Nonanolen (durch Hydroformylierung und Hydrierung), die u. a. als Weichmacheralkohole genutzt werden.

Die aus dem Reaktionsaustrag abgetrennte C₄-Fraktion kann auf verschiedene Arten aufgearbeitet werden. Zum einen kann aus der C₄-Fraktion zunächst das 1,2-Butadien, z. B. destillativ abgetrennt werden und einer weiteren Verwendung zugeführt werden. Zum anderen kann die C₄-Fraktion einer Selektiv-Hydrierung zugeführt werden, in welcher die Diene entfernt werden, also Reste an 1,3-Butadien und das 1,2-Butadien zu 1-Buten und 2-Butenen umgesetzt werden. Derartige Hydrierungen sind aus dem Stand der Technik bekannt und werden beispielsweise in US 5475173, DE 3119850 und F. Nierlich, F. Obenhaus, Erdöl & Kohle, Erdgas, Petrochemie (1986) 39, 73 - 78 beschrieben. Technisch werden sie sowohl einals auch mehrstufig ausgeführt. Bevorzugt erfolgt die Hydrierung in der Flüssigphase an heterogenen Palladium-Trägerkatalysatoren. Der in der C₄-Fraktion gegebenenfalls vorhandene Alkohol kann, falls notwenig, vor oder nach der Hydrierung mittels bekannter Verfahren abgetrennt werden. Leicht in Wasser lösliche Alkohole (beispielsweise Isopropanol) können z. B. über eine Wasserwäsche entfernt werden. Zur Trocknung des C₄-Stroms haben sich unter anderem Trocknungskolonnen bewährt. Die so erhaltene Mischung der weitgehend 1,3-Butadien-, 1,2-Butadien- und Alkohol- freien C₄-Kohlenwaserstoffe (Butadiengehalt vorzugsweise kleiner 5000 ppm) entspricht weitestgehend handelsüblichem Raffinat I und kann entsprechend den bekannten Verfahren wie Raffinat I weiterverarbeitet bzw. aufgearbeitet werden. Beispielsweise kann es zur Herstellung von tert.-Butylalkohol, Diisobuten (oder Isooktan), Methyl-tert.-butylether, 1-Buten oder C₄-Di- und Oligomeren eingesetzt werden.

Die folgenden Beispiele sollen die Erfindung näher erläutern, ohne den Schutzbereich zu beschränken, der sich aus der Beschreibung und den Patentansprüchen ergibt.

### Beispiel 1

0,005 mol eines 1,3-Bis-(2,6-diisopropylphenyl)-4,5-dimethyl-3H-imidazolidenyl-Palladium(0)-DVDS-Komplexes (Komplex aus Palladium, dem Liganden L1 und DVDS als weiteren Liganden) wurden in 33,3 g einer 0,5 molaren Lösung von Natriumisopropylat in Isopopanol gelöst. Das Gemisch wurde unter Argon in einen rostfreien 100 ml großen Parr-Autoklaven gefüllt. Der Autoklav wurde mit Hilfe eines Kühlbad auf -15 °C gekühlt. Anschließend wurden aus einem Druckzylinder unter Massenkontrolle 15,0 g (2,77 /10⁻¹ mol) 1,3-Butadien in den Autoklaven kondensiert. Nach Verschließen des Autoklavens wurde auf 70 °C aufgeheizt und die Temperatur 16 h konstant gehalten. Dann wurde der Autoklav auf Raumtemperatur abgekühlt. Das bei der Druckentspannung entweichende Butadien wurde kondensiert und ausgewogen. Zu dem verbleibenden Autoklaveninhalt wurden 5 g Isooctan als innerer Standard gegeben. Die Ausbeute an linearen Octatrien wurde gaschromatrographisch unter Verwendung des Geräts HP 6869 A bestimmt. Die Ausbeute an linearen 1,3,7-Octatrienen betrug 92 % bei einer Chemoselektivität von 95 %. Mit Chemoselektivität gleich Ausbeute an Octatrienen multipliziert mit 100 geteilt durch die Summe der Ausbeuten an Telomer, Octatrienen und 4-Vinylcyclohexen.

### Beispiel 2

Beispiel 2 wurde analog Beispiel 1 durchgeführt mit dem Unterschied, dass der sekundäre Alkohol Cyclohexanol und die Base Natriumcyclohexanolat war. Die Ausbeute an linearen 1,3,7-Octatrienen betrug 83 % bei einer Chemoselektivität von 95 %.

### Beispiel 3

Beispiel 3 wurde analog Beispiel 1 durchgeführt mit dem Unterschied, dass an Stelle des Komplexes 1,3-Bis (2,6-diisopropylphenyl)-4,5-dimethyl-3-hydroimidazoliumbromid und 0,005 mol% Palladiumacetat (1,38 * 10⁻⁵ mol) im Verhältnis von 4 zu 1 eingesetzt wurden. Die Ausbeute an linearen 1,3,7-Octatrienen betrug 83 % bei einer Chemoselektivität von 93 %.

## Patentansprüche

1. Verfahren zur Herstellung von linearen Octatrienen, aus 1,3-Butadien oder 1,3-Butadien enthaltenden Kohlenwasserstoffgemischen,
**dadurch gekennzeichnet,**
**dass** die Dimerisierung von 1,3-Butadien in Gegenwart eines sekundären Alkohols und einer Base erfolgt und dass als Katalysator ein Komplex eines Metalls der achten Nebengruppe des Periodensystems der Elemente verwendet wird, der als Ligand zumindest ein Carben mit der Struktur L aufweist, 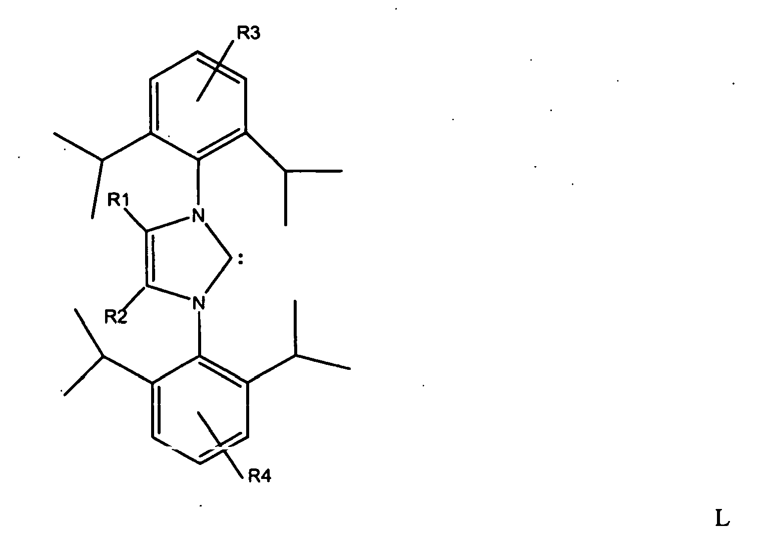 mit R1 und R2 = C₁- bis C₃-Alkylrest und R3 und R4 = H oder C₁- bis C₃-Alkylrest, wobei die Reste R1 und R2 bzw. R3 und R4 gleich oder unterschiedlich sein können.

2. Verfahren nach Anspruch 1,
**dadurch gekennzeichnet.**
**dass** der Katalysator als Carben-Ligand 1,3-Bis-(2,6-diisopropylphenyl)-4,5-dimethyl-2-dehydro-3-hydro-imidazol (Strukturformel L1) aufweist.

3. Verfahren nach Anspruch 1 oder 2,
**dadurch gekennzeichnet.**
**dass** ein Katalysator eingesetzt wird, der als Metall Palladium oder Nickel aufweist.

4. Verfahren nach zumindest einem der vorherigen Ansprüche,
**dadurch gekennzeichnet.**
**dass** als Base Alkoholate verwendet werden.

5. Verfahren nach zumindest einem der vorherigen Ansprüche,
**dadurch gekennzeichnet,**
**dass** Alkalimetallhydroxide als Basen eingesetzt werden.

6. Verfahren nach zumindest einem der vorherigen Ansprüche,
**dadurch gekennzeichnet,**
**dass** als sekundärer Alkohol Isopropanol verwendet wird.

7. Verfahren nach zumindest einem der vorherigen Ansprüche,
**dadurch gekennzeichnet,**
**dass** als sekundärer Alkohol Cyclohexanol verwendet wird.

8. Verfahren nach zumindest einem der vorherigen Ansprüche,
**dadurch gekennzeichnet,**
**dass** das Carben mit der Struktur L und der daraus abgeleitete Metallkomplex in situ aus einem Imidazoliumsalz der allgemeinen Struktur S 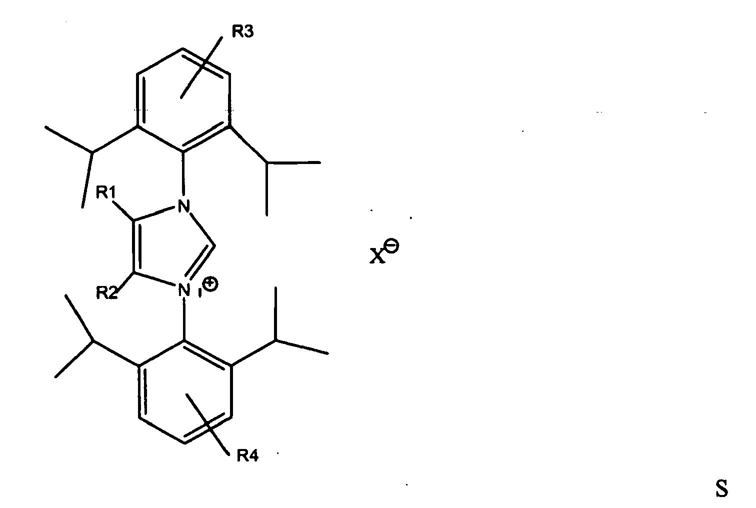 mit R1 und R2 = C₁- bis C₃-Alkylrest und R3 und R4 = H oder C₁- bis C₃-Alkylrest, wobei die Reste R1 und R2 bzw. R3 und R4 gleich oder unterschiedlich sein können, und einer Metallverbindung erzeugt wird.

9. Verfahren nach Anspruch 8,
**dadurch gekennzeichnet,**
**dass** das Carben 1,3-Bis-(2,6-diisopropylphenyl)-4,5-dimethyl-2-dehydro-3-hydro-imidazol und der daraus abgeleitete Metallkomplex in situ aus einem 1,3-Bis-(2,6-diisopropylphenyl)-4,5-dimethyl-3-hydro-imidazoliumsalz mit der allgemeinen Struktur S1 und einer Metallverbindung erzeugt wird.

10. Verfahren nach einem der vorherigen Ansprüche,
**dadurch gekennzeichnet,**
**dass** als Edukt ein Butadien-haltiger C₄-Schnitt verwendet wird.

11. Verfahren nach einem der vorherigen Ansprüche,
**dadurch gekennzeichnet,**
**dass** die Konzentration des Katalysators, formal angegeben in ppm (Masse) an Metall bezogen auf die Gesamtmasse, von 0,01 ppm bis 1000 ppm beträgt.

12. Verfahren nach einem der vorherigen Ansprüche,
**dadurch gekennzeichnet,**
**dass** das Verhältnis (Mol/Mol) von Carben L zu Metall im Bereich von 0,01/1 bis 250/1 eingestellt wird.

13. Verfahren nach einem der vorherigen Ansprüche,
**dadurch gekennzeichnet,**
**dass** neben dem Carben-Liganden zumindest ein weiterer Ligand vorhanden ist.

14. Verfahren nach Anspruch 13,
**dadurch gekennzeichnet,**
**dass** neben dem Carben-Liganden zumindest 1,1,3,3-Tetramethyl-1,3-Divinyldisiloxan als ein weiterer Ligand vorhanden ist.

15. Verfahren nach Anspruch 13 oder 14,
**dadurch gekennzeichnet,**
**dass** das Verhältnis der weiteren Liganden zu dem Carbenliganden L vorzugsweise von 0,1 zu 1 bis 10 zu 1 beträgt.

16. Carbenkomplexkatalysator,
**dadurch gekennzeichnet,**
**dass** der Katalysator ein Ligandenkomplex eines Metalls der achten Nebengruppe des Periodensystems der Elemente ist, der als Ligand zumindest ein Carben mit der Struktur L aufweist, 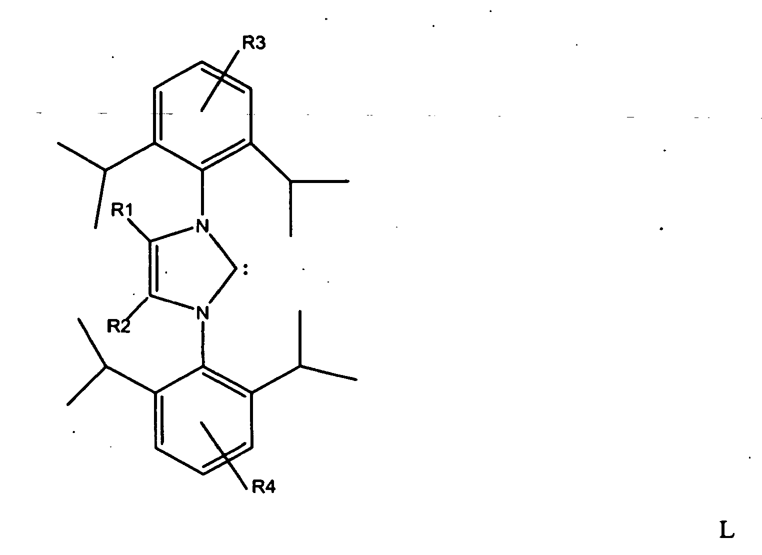 mit R1 und R2 = C₁- bis C₃-Alkylrest und R3 und R4 = H oder C₁- bis C₃-Alkylrest, wobei die Reste R1 und R2 bzw. R3 und R4 gleich oder unterschiedlich sein können.

## Claims

1. Process for preparing linear octatrienes from 1,3-butadiene or 1,3-butadiene-containing hydrocarbon mixtures,
**characterized in that**
the dimerization of 1,3-butadiene is carried out in the presence of a secondary alcohol and a base, and a complex of a metal of transition group eight of the Periodic Table of the Elements having at least one carbene of the structure L 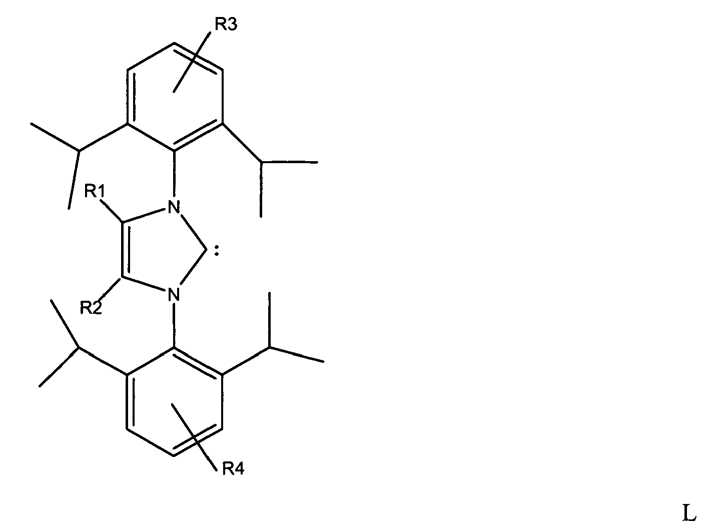 where R1 and R2 = C₁-C₃-alkyl radical and R3 and R4 = H or C₁-C₃-alkyl radical, with the radicals R1 and R2 or R3 and R4 being able to be identical or different, as ligand is used as catalyst.

2. Process according to Claim 1,
**characterized in that**
the catalyst comprises 1,3-bis(2,6-diisopropylphenyl)-4,5-dimethyl-2-dehydro-3-hydroimidazole (structural formula L1) as carbene ligand.

3. Process according to Claim 1 or 2,
**characterized in that**
a catalyst having palladium or nickel as metal is used.

4. Process according to at least one of the preceding claims,
**characterized in that**
alkoxides are used as base.

5. Process according to at least one of the preceding claims,
**characterized in that**
alkali metal hydroxides are used as bases.

6. Process according to at least one of the preceding claims,
**characterized in that**
isopropanol is used as secondary alcohol.

7. Process according to at least one of the preceding claims,
**characterized in that**
cyclohexanol is used as secondary alcohol.

8. Process according to at least one of the preceding claims,
**characterized in that**
the carbene of the structure L and the metal complex derived therefrom are generated in situ from an imidazolium salt of the general structure S 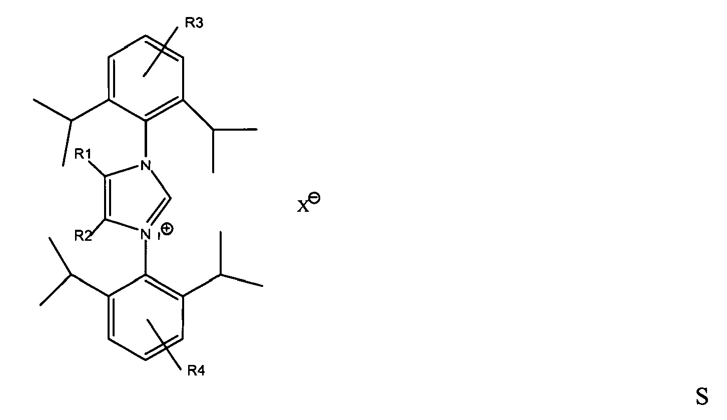 where R1 and R2 = C₁-C₃-alkyl radical and R3 and R4 = H or C₁-C₃-alkyl radical, with the radicals R1 and R2 or R3 and R4 being able to be identical or different, and a metal compound.

9. Process according to Claim 8,
**characterized in that**
the carbene 1,3-bis(2,6-diisopropylphenyl)-4,5-dimethyl-2-dehydro-3-hydroimidazole and the metal complex derived therefrom are generated in situ from a 1,3-bis(2,6-diisopropylphenyl)-4,5-dimethyl-3-hydroimidazolium salt of the general structure S1 and a metal compound.

10. Process according to any of the preceding claims,
**characterized in that**
a butadiene-containing C₄ fraction is used as starting material.

11. Process according to any of the preceding claims,
**characterized in that**
the concentration of the catalyst, formally reported in ppm (mass) of metal based on the total mass, is from 0.01 ppm to 1000 ppm.

12. Process according to any of the preceding claims,
**characterized in that**
the ratio (mol/mol) of carbene L to metal is set in the range from 0.01/1 to 250/1.

13. Process according to any of the preceding claims,
**characterized in that**
at least one further ligand is present in addition to the carbene ligand.

14. Process according to Claim 13,
**characterized in that**
at least 1,1,3,3-tetramethyl-1,3-divinyldisiloxane is present as a further ligand in addition to the carbene ligand.

15. Process according to Claim 13 or 14,
**characterized in that**
the ratio of the further ligands to the carbene ligand L is preferably from 0.1 : to10:1.

16. Carbene complex catalyst,
**characterized in that**
the catalyst is a ligand complex of a metal of transition group eight of the Periodic Table of the Elements which has at least one carbene of the structure L, 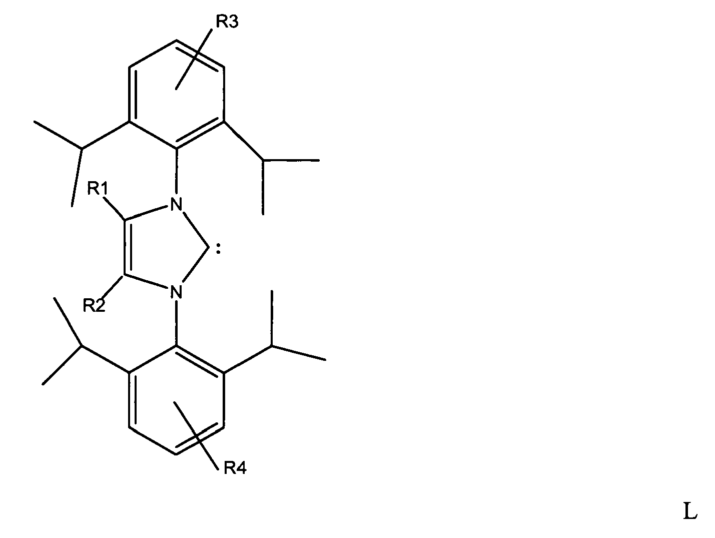 where R1 and R2 = C₁-C₃-alkyl radical and R3 and R4 = H or C₁-C₃-alkyl radical, with the radicals R1 and R2 or R3 and R4 being able to be identical or different, as ligand.

## Revendications

1. Procédé pour la préparation d'octatriènes linéaires, à partir de 1,3-butadiène ou de mélanges d'hydrocarbures contenant du 1,3-butadiène, **caractérisé en ce qu'**on effectue la dimérisation du 1,3-butadiène en présence d'un alcool secondaire et d'une base, et on utilise comme catalyseur un complexe d'un métal du groupe VIIIB du système périodique des éléments, qui comporte en tant que ligand au moins un carbène ayant la structure L, 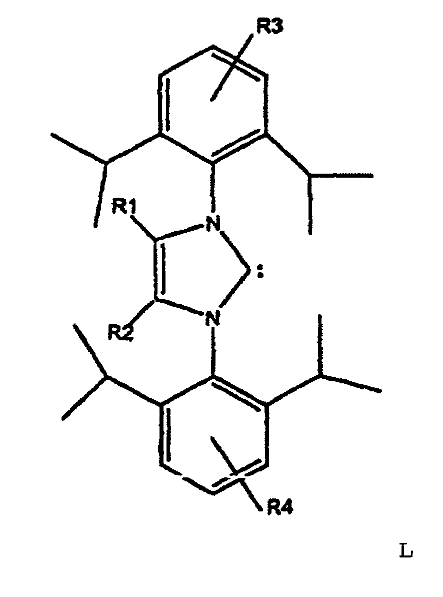 où R1 et R2 représentent un radical alkyle en C₁-C₃ et R3 et R4 = H ou un radical alkyle en C₁-C₃, les radicaux R1 et R2 ou, respectivement, R3 et R4, pouvant être identiques ou différents.

2. Procédé selon la revendication 1, **caractérisé en ce que** le catalyseur comporte en tant que ligand carbène du 1,3-bis(2,6-diisopropylphényl)-4,5-diméthyl-2-déhydro-3-hydro-imidazole (formule développée L1).

3. Procédé selon la revendication 1 ou 2, **caractérisé en ce qu'**on utilise un catalyseur qui comporte en tant que métal du palladium ou du nickel.

4. Procédé selon au moins l'une des revendications précédentes, **caractérisé en ce qu'**on utilise comme base des alcoolates.

5. Procédé selon au moins l'une des revendications précédentes, **caractérisé en ce qu'**on utilise comme bases des hydroxydes de métaux alcalins.

6. Procédé selon au moins l'une des revendications précédentes, **caractérisé en ce qu'**on utilise comme alcool secondaire l'isopropanol.

7. Procédé selon au moins l'une des revendications précédentes, **caractérisé en ce qu'**on utilise comme alcool secondaire le cyclohexanol.

8. Procédé selon au moins l'une des revendications précédentes, **caractérisé en ce que** le carbène ayant la structure L et le complexe métallique dérivé de celui-ci sont formés in situ à partir d'un sel d'imidazolium de structure générale S 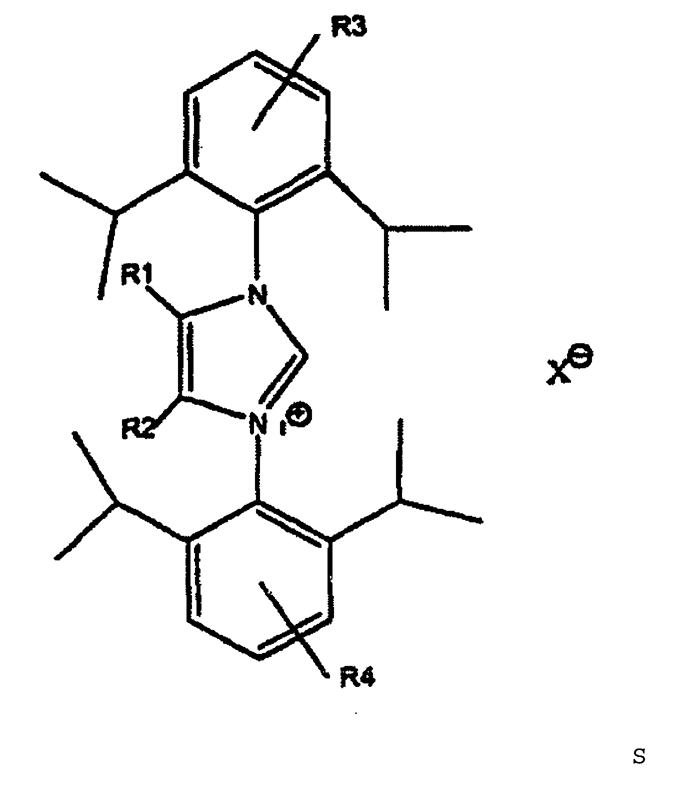 où R1 et R2 représentent un radical alkyle en C₁-C₃ et R3 et R4 = H ou un radical alkyle en C₁-C₃, les radicaux R1 et R2 ou, respectivement, R3 et R4, pouvant être identiques ou différents, et d'un composé métallique.

9. Procédé selon la revendication 8, **caractérisé en ce que** le carbène 1,3-bis(2,6-diisopropylphényl)-4,5-diméthyl-2-déhydro-3-hydro-imidazole et le complexe métallique dérivé de celui-ci sont formés in situ à partir d'un sel de 1,3-bis(2,6-diisopropylphényl)-4,5-diméthyl-3-hydroimidazolium ayant la structure générale S1 et d'un composé métallique.

10. Procédé selon l'une quelconque des revendications précédentes, **caractérisé en ce qu'**on utilise comme produit de départ une fraction en C₄ contenant du butadiène.

11. Procédé selon l'une quelconque des revendications précédentes, **caractérisé en ce que** la concentration du catalyseur, indiquée selon la formule en ppm (masse) de métal par rapport à la masse totale, va de 0,01 ppm à 1 000 ppm.

12. Procédé selon l'une quelconque des revendications précédentes, **caractérisé en ce que** le rapport [mole(s)/mole] du carbène L au métal est ajusté dans la plage de 0,01/1 à 250/1.

13. Procédé selon l'une quelconque des revendications précédentes, **caractérisé en ce qu'**au moins un autre ligand est présent en plus du ligand carbène.

14. Procédé selon la revendication 13, **caractérisé en ce qu'**au moins du 1,1,3,3-tétraméthyl-1,3-divinyldisiloxane est présent en tant qu'autre ligand en plus du ligand carbène.

15. Procédé selon la revendication 13 ou 14, **caractérisé en ce que** le rapport de l'autre ligand au ligand carbène L va de préférence de 0,1:1 à 10:1.

16. Catalyseur de type complexe de carbène, **caractérisé en ce que** le catalyseur est un complexe de ligand d'un métal du groupe VIIIB du système périodique des éléments, qui comporte en tant que ligand au moins un carbène ayant la structure L, 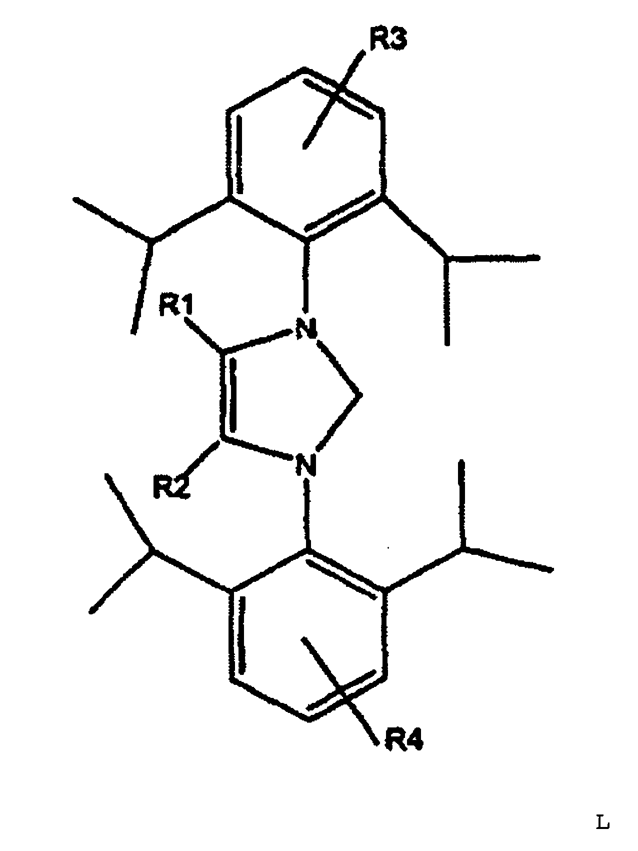 où R1 et R2 représentent un radical alkyle en C₁-C₃ et R3 et R4 = H ou un radical alkyle en C₁-C₃, les radicaux R1 et R2 ou, respectivement, R3 et R4, pouvant être identiques ou différents.
